# EUROPEAN PATENT SPECIFICATION

(11) **EP 3 175 842 B1**
(45) Date of publication and mention of the grant of the patent: **11.12.2019**
(21) Application number: 15197874.9
(22) Date of filing: 03.12.2015
(51) Int. Cl.: A61K 9/00, A61K 31/439, A61K 9/14

(54) **DRY POWDER MIXING PROCESS**
TROCKENPULVERMISCHVERFAHREN
PROCÉDÉ DE MÉLANGE DE POUDRE SÈCHE

(43) Date of publication of application: 07.06.2017
(73) Proprietor: Alfred E. Tiefenbacher (GmbH & Co. KG), 22767 Hamburg (DE); Naonopharm Ltd., Bath, Somerset BA1 2PA (GB)
(72) Inventor: Meyer, Claudia, 22767 Hamburg (DE); Price, Robert, Chepstow, NP16 6LW (GB); Shur, Jagdeep, Bath (GB)
(74) Representative: Hamm&Wittkopp Patentanwälte PartmbB

(56) References cited:
- WO-A1-2013/109214
- US-A1- 2002 106 332

## Description

The present invention relates to a process for preparing a dry powder formulation suitable for inhalation containing tiotropium bromide and a lactose carrier.

Powder formulations for inhalation are typically administered by means of dry powder inhalers (DPIs). Several types of DPI devices exist such as reservoir devices, capsule devices and blister devices. In a typical device the powder constitutes of a relatively large amount of excipient with a small amount of drug, typically in the order of 1-2 % of the loading. In order to deposit the drug particles in the deep lungs, the mass median aerodynamic diameter (MMAD) of the drug particles is 10 µm or less, typically in the range of 0.5-5 µm.

The larger excipient particles are responsible for the flow properties of the dry powder inhalation formulation when portioning the inhalation powder in single doses, e.g. when filling into capsules or upon loading a dose into the reservoir of the inhalation device, because due to the low amount of drug powder bulk properties, such as powder flow, are expected to rely largely on the excipient properties. Moreover, powder bulk properties also play a major role in the inhalation process. Dry powder inhalation formulations are typically prepared by blending the drug and the excipient. During the mixing process the drug particles are adhered to the excipient particles. The excipient particles thus function as carrier particles, and it is important that the drug may be detached from the carrier particles during inhalation. Therefore, on the one hand, the interaction between drug and carrier should be strong, so that the generation of agglomerates of drug particles is prevented. On the other hand, the interaction should be weak enough to be broken during the inhalation event. A suitable balance of these properties is provided by lactose, and various lactose grades are commercially available for the preparation of dry powder inhalation formulations.

Hence, providing good flow characteristics of the dry powder inhalation formulation and an appropriate adherence of the drug to the carrier particles are of great importance. Moreover, the uniformity of the dry powder inhalation formulation is of great importance, too. Content uniformity largely depends on the mixing process used for the preparation of the dry powder inhalation formulation. Particularly, the content uniformity is a crucial factor in highly active drug formulations. Those formulations typically contain a very small proportion of active ingredient to deliver single doses in the microgram range, or even less. Examples of those highly active substances are anticholinergic compounds like Ipratropium bromide or Tiotropium bromide and β-sympathomimetics like Formoterol or Salmeterol.

Flow characteristics, adherence of the drug on the carrier particles and content uniformity eventually serve the purpose of ensuring dosing accuracy. Various techniques are known in the art for providing above characteristics of the dry powder inhalation formulation.

WO 93/11746 reports that coarse excipient particles provide good flow characteristics but negatively influence the liberation of the drug in the lungs. In order to overcome this problem a mixture of coarse excipient particles having an average particle size of 20 µm or more and fine excipient particles having an average particle size of 10 µm or less is used.

According to DE 44 25 255 a high bulk density of the dry powder inhalation formulation and similar bulk and tapped densities are decisive factors for achieving good flow characteristics. It was found that mixing a micronized drug with excipient particles having an average particle size in the range of 200 µm to 1,000 µm, preferably, 300 µm to 600 µm, provides spherical carrier particles with the drug deposited on the surface.

WO 2013/109214 describes a process for preparing a dry powder inhalation formulation containing budesonide. In this process, the main part of the coarse excipient is divided into four equal fractions, and the remaining part of the coarse excipient is mixed with the fine excipient to obtain a fine excipient/coarse excipient mixture. Budesonide is mixed with one fraction of coarse excipient to obtain a budesonide/ coarse excipient mixture. The dry powder inhalation formulation is then prepared as follows. Two fractions of coarse excipient are placed in a blender. Subsequently, the budesonide/coarse excipient mixture, the fine excipient/coarse excipient mixture and finally the fourth fraction of coarse excipient are added. Blending the four-layered composition gives the final dry powder inhalation formulation.

According to the process described in WO 2004/017918 a first portion of the drug is mixed with an excipient to obtain a first preblend that is subsequently mixed with a second drug to form a second preblend. Finally, the second preblend is mixed with the remaining portion of the first drug to obtain the dry powder inhalation formulation.

WO 2007/068443 describes a process for the preparation of a dry powder inhalation formulation, in which a drug is mixed with a force-controlling agent, e.g. a phospholipids, titanium dioxide, aluminium dioxide, silicon dioxide, starch or salts or fatty acids, in order to obtain drug particles having the force-controlling agent deposited on the surface. This composite material is subsequently mixed with a small portion of the carrier material to obtain a preblend that is subsequently mixed with the remaining portion of the carrier material. The force-controlling agent serves the purpose to adjust the interaction between drug and carrier.

EP 1 326 585 discloses a mixing process, in which the drug and the excipient are divided into substantially equal fractions. These fractions are placed in alternate layers in a suitable mixer, whereby the excipient is placed in the mixer first. A relatively high content uniformity is obtained if at least 10, preferably 20, and even more preferably more than 30 layers are put together in the mixer to form the composition.

Hence, various methods have been suggested in the art to prepare a dry powder inhalation formulation showing good flow characteristics and content uniformity. These methods may be classified as dilution methods, in which a drug/carrier preblend is subsequently diluted with additional carrier, or as multiple-layer mixing processes, in which the entire drug and the entire excipient are placed in a mixer as alternate, equal layers and subsequently blended.

They suffer, however, from several setbacks, as shown in example 1 of EP 1 326 585. In a first step, 33.5 kg of a lactose preblend was prepared by 31 and 30 layers of coarse and fine lactose, respectively, which were subsequently mixed in a free fall mixer. In a second step, a total of 32.87 kg of the lactose preblend was then mixed with 0.13 kg micronized Tiotropium bromide monohydrate. The excipient was thereby added in 46 layers, the active ingredient in 45 layers, and subsequently mixed in a free fall mixer, to ensure a proper content uniformity of the mixture.

It can easily be recognized that a mixing process requiring a total of 152 weighting steps is extremely unfavourable. Furthermore, the very small amount of the single Tiotropium bromide monohydrate layer (0.003 kg) compared to the overall scale of the preparation will cause additional handling problems.

It was an objective of the present invention to provide a process for preparing a dry powder inhalation formulation showing excellent flow characteristics and content uniformity by a simple, robust and effective process.

This objective is attained by the subject matter as defined in the claims.

The present invention relates to a process for preparing a dry powder inhalation formulation containing tiotropium bromide and a lactose carrier, comprising the steps:
a) splitting the lactose carrier into a first, a second and a third portion,
b) mixing the first and the second portion of the lactose carrier and the drug by
   i) placing the first portion of the lactose carrier in a mixer to obtain a first layer,
   ii) placing the entire drug on top of the first lactose layer to obtain a second layer,
   iii) placing the second portion of the lactose carrier on top of the second layer to obtain a third layer,
   iv) blending the three-layered composition obtained in step (iii) to obtain a preblend,
   v) optionally sifting the preblend,
c) mixing the preblend obtained in step (b) and the third portion of the lactose carrier to obtain the dry powder inhalation formulation, and
d) optionally sifting the dry powder inhalation formulation.

The particle size of the lactose carrier used in the dry powder inhalation formulation must be suitable to yield good flowability. The particle size is typically determined by laser diffraction (Sympatec or Malvern). Various grades of lactose suitable for inhalation are commercially available (e.g. Lactohale®, DFE Pharma). According to a preferred embodiment of the present invention, the lactose carrier is prepared by mixing coarse lactose and fine lactose. While coarse lactose improves the flowability of the dry powder inhalation formulation, fine lactose (including micronized lactose) allows uniform attachment of the one or more drugs to the carrier particles. Coarse lactose has a volume median diameter (VMD) typically in the range of 50-150 µm, while the VMD of fine lactose is typically below 50 µm, usually in the range of 15-30 µm. Micronized lactose has a VMD of 10 µm or less, typically 5 µm or less. Preferably, the fine lactose portion of the lactose carrier is 30% by weight or less, more preferred 10-30% by weight and most preferred 20-30% by weight.

The process of the present invention is suitable for preparing a dry powder inhalation formulation containing Tiotropium bromide, and preferably Tiotropium bromide monohydrate.

It is preferred that the lactose carrier is prepared in the same way as the dry powder inhalation formulation, i.e. by a process comprising the steps:
a1) splitting the coarse lactose into a first, a second and a third fraction,
a2) placing the first fraction of the coarse lactose in a mixer to obtain a first layer,
a3) placing the entire fine lactose on top of the first layer to obtain a second layer,
a4) placing the second fraction of the coarse lactose on top of the second layer to obtain a third layer,
a5) blending the three-layered composition obtained in step (a4) to obtain a preblend,
a6) optionally sifting the preblend,
a7) mixing the preblend obtained in step (a5) or (a6) and the third fraction of the coarse lactose to obtain the lactose carrier, and
a8) optionally sifting the lactose carrier.

Preferably, the first, second and third fractions of the coarse lactose in the preparation of the lactose carrier are equal.

According to a preferred embodiment of the present invention, the amount of the first portion, the second portion and the third portion of the lactose carrier in the preparation of the dry powder inhalation formulation each is at least 10 % by weight, based on the total weight of the lactose carrier. It is even more preferred that the amounts of the respective portions of the lactose carrier are not equal. It has been found that excellent content uniformity was achieved if the weight ratio first portion/second portion/third portion of the lactose carrier is 7/2/1, 5/1/4, 5/2/3, 4/1/5, or 2/1/7. Preferably, the mixing device for preparing the lactose carrier as well as the dry powder inhalation formulation of the present invention is a Turbula® mixer.

According to a preferred embodiment of the present invention, the dry powder inhalation formulation consists of tiotropium bromide, preferably tiotropium bromide monohydrate, and the lactose carrier, preferably lactose monohydrate.

The following examples are intended to further illustrate the present invention.

### Examples

Content uniformity as expressed by the target content per dosage unit (% Assay), the relative standard deviation (% RSD) and the acceptance value (AV) is determined according to USP 38 <905>.

### Example 1. Manufacture of lactose carrier

1. Pre-sieving
   1.1. Pre-sieve fine lactose through a 250 µm sieve.
   1.2. Pre-sieve coarse lactose through a 250 µm sieve.
2. Lactose carrier manufacture
   - Add 1/3 of the coarse lactose, add all fine lactose, add approximately 1/3 of the coarse lactose.
   - Seal the mixing jar and mix on the Turbula mixer at 22 rpm for 15 minutes.
   - Pass the mixture through 250 µm sieve
   - Add remaining 1/3 of the coarse lactose.
   - Seal the mixing jar and mix on the Turbula mixer at 22 rpm for 15 minutes.
   - Once blending is completed, sieve the lactose carrier through 250 µm sieve and collect.

The fine lactose content of the lactose carrier is 25% by weight.

Figure 1 below displays this process as a flow diagram.

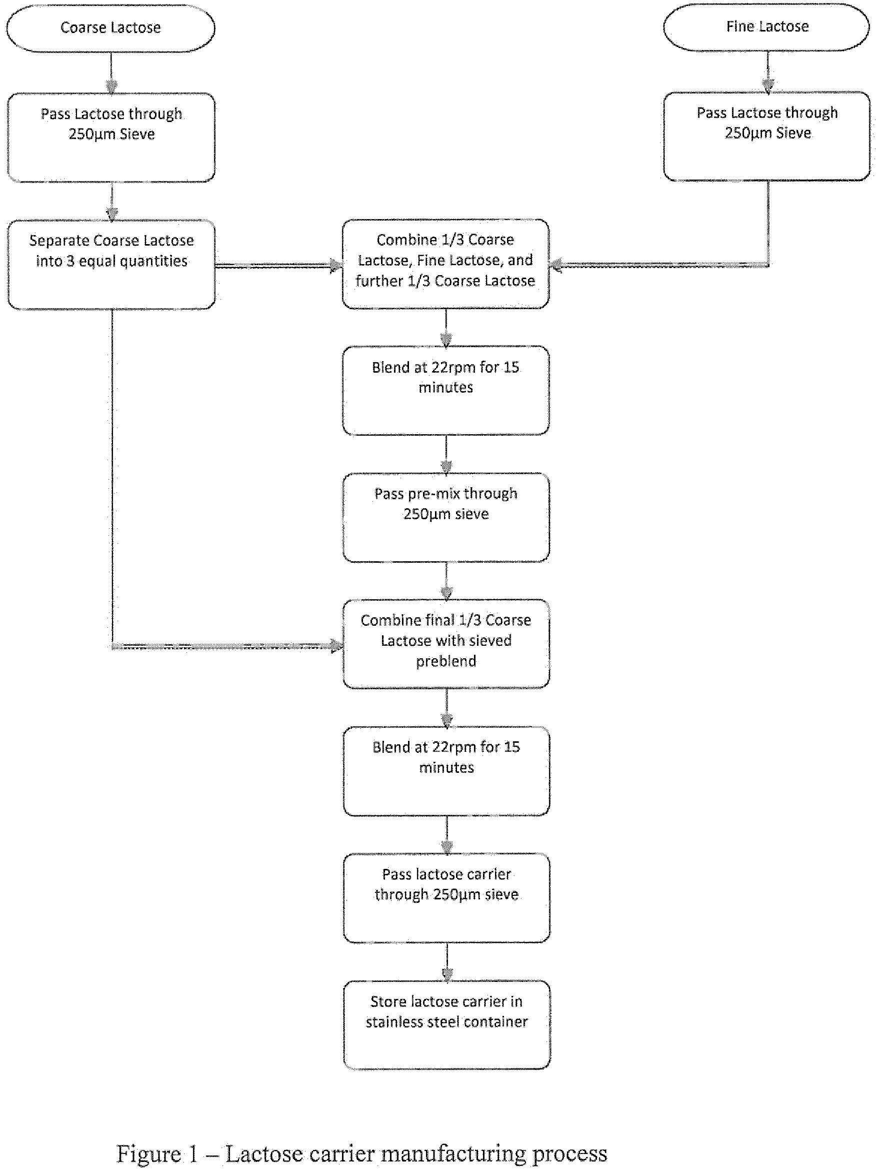

### Examples 2-6 Preparation of dry powder inhalation formulation containing Tiotropium bromide monohydrate (nominal concentration 0.41 % w/w active ingredient)

1. Pre-sieving
   1.1. Pre-sieve lactose carrier through a 250 µm sieve.
   1.2. Pre-sieve tiotropium bromide monohydrate through a 250 µm sieve.
2. Layer Preparation
   2.1. Separate the lactose carrier into 10 equal quantities.
3. Formulation Construction
   3.1. Add **5 quantities** of lactose carrier into the blending vessel.
   3.2. Add the **whole amount** of tiotropium bromide monohydrate into the blending vessel directly on top of the lactose.
   3.3. Add **2 quantities** of lactose carrier into the vessel directly on top of the tiotropium bromide monohydrate.
   3.4. Seal the vessel and place into the Turbula mixer at 22rpm for 15 minutes.
   3.5. Add final **3 quantities** of lactose carrier on top of the blend.
   3.6. Reseal the vessel, place into the Turbula mixer at 22rpm for further 15 minutes.
   3.7. Remove the vessel and pass the formulation through a 250 µm sieve.

Figure 2 displays this process as a flow diagram.

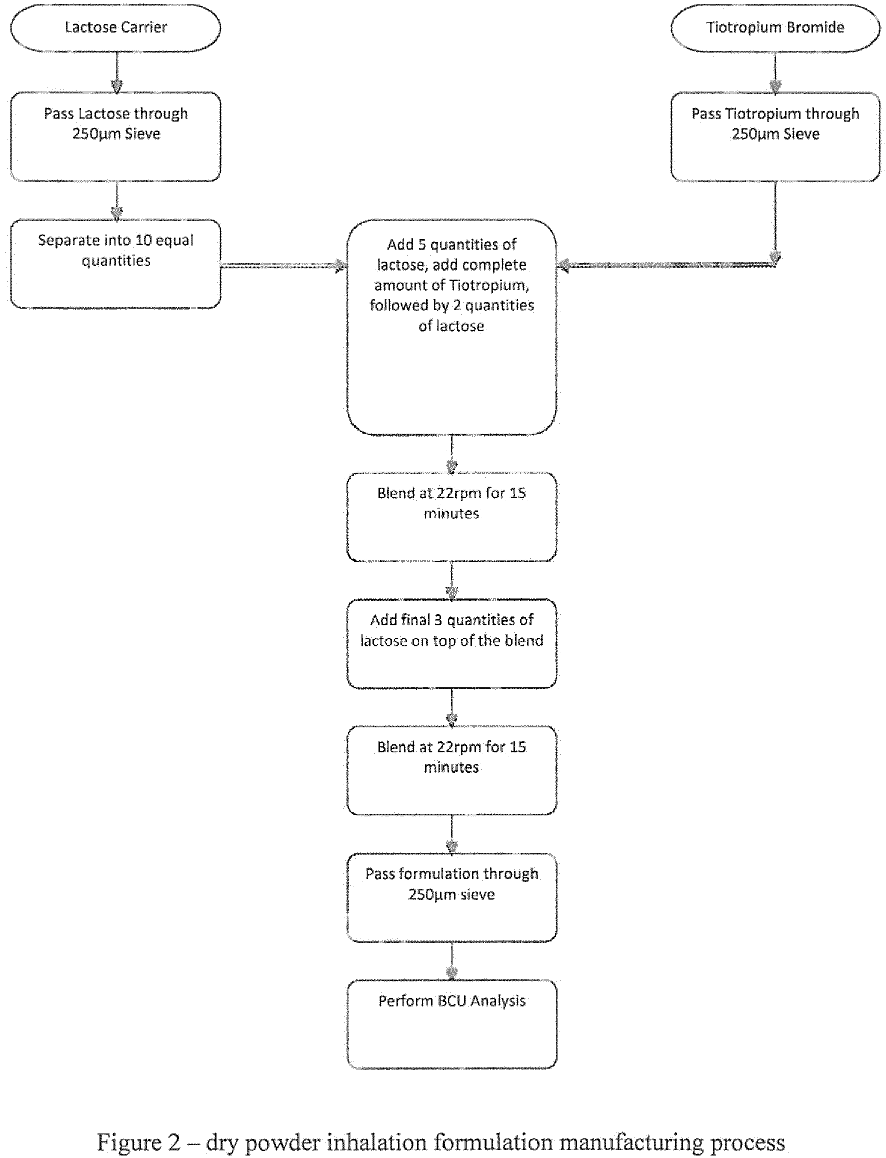

Above process was repeated with the following weight ratios first portion/second portion/third portion of the lactose carrier: 7/2/1, 5/1/4, 4/1/5 and 2/1/7.

**Table 1. Weight ratio of first portion/second portion/third portion of the lactose carrier**

| **Ex.** | **1^{st} Blending (step b)** | | **2^{nd} Blending (step c)** |
|---|---|---|---|
| 2 | 5 Parts Lactose Carrier | 2 Parts Lactose Carrier | 3 Part Lactose Carrier |
| 3 | 7 Parts Lactose Carrier | 2 Parts Lactose Carrier | 1 Part Lactose Carrier |
| 4 | 5 Parts Lactose Carrier | 1 Part Lactose Carrier | 4 Parts Lactose Carrier |
| 5 | 4 Parts Lactose Carrier | 1 Part Lactose Carrier | 5 Parts Lactose Carrier |
| 6 | 2 Parts Lactose Carrier | 1 Part Lactose Carrier | 7 Parts Lactose Carrier |

**Table 2. Content uniformity**

| **Ex.** | **% Assay** | **% RSD** | **AV** |
|---|---|---|---|
| 2 | 99.9 | 3.3 | 7.8 |
| 3 | 100.4 | 2.9 | 7.1 |
| 4 | 102.2 | 4.8 | 12.5 |
| 5 | 101.0 | 3.2 | 7.7 |
| 6 | 101.5 | 4.1 | 10.0 |

## Claims

1. Process for preparing a dry powder inhalation formulation containing tiotropium bromide and a lactose carrier, comprising the steps:
a) splitting the lactose carrier into a first, a second and a third portion,
b) mixing the first and the second portion of the lactose carrier and the drug by
i) placing the first portion of the lactose carrier in a mixer to obtain a first layer,
ii) placing the entire drug on top of the first lactose layer to obtain a second layer,
iii) placing the second portion of the lactose carrier on top of the second layer to obtain a third layer,
iv) blending the three-layered composition obtained in step (iii) to obtain a preblend,
v) optionally sifting the preblend,
c) mixing the preblend obtained in step (b) and the third portion of the lactose carrier to obtain the dry powder inhalation formulation, and
d) optionally sifting the dry powder inhalation formulation.

2. Process according to claim 1, wherein the lactose carrier is prepared by mixing coarse lactose and fine lactose.

3. Process according to claim 2, comprising the steps:
a1) splitting the coarse lactose into a first, a second and a third fraction,
a2) placing the first fraction of the coarse lactose in a mixer to obtain a first layer,
a3) placing the entire fine lactose on top of the first layer to obtain a second layer,
a4) placing the second fraction of the coarse lactose on top of the second layer to obtain a third layer,
a5) blending the three-layered composition obtained in step (a4) to obtain a preblend,
a6) optionally sifting the preblend,
a7) mixing the preblend obtained in step (a5) or (a6) and the third fraction of the coarse lactose to obtain the lactose carrier, and
a8) optionally sifting the lactose carrier.

4. Process according to claim 3, wherein the first, second and third fractions of the coarse lactose are equal.

5. Process according to any one of claims 2 to 4, wherein the fine lactose content of the lactose carrier is 30% by weight or less, preferably 10-30% by weight, more preferred 20-30% by weight.

6. Process according to any one of the preceding claims, wherein the amount of the first portion, the second portion and the third portion of the lactose carrier each is at least 10 % by weight, based on the total weight of the lactose carrier.

7. Process according to claim 6, wherein the amount of the first portion of the lactose carrier is 10-70% by weight, the amount of the second portion of the lactose carrier is 10-20% by weight, and the amount of the third portion of the lactose carrier is 10-70% by weight, based on the total weight of the lactose carrier.

8. Process according to claim 6 or 7, wherein the weight ratio of the first portion/second portion/third portion of the lactose carrier is 7/2/1, 5/2/3, 5/1/4, 4/1/5 or 2/1/7.

9. Process according to any one of the preceding claims, wherein the dry powder inhalation formulation consists of tiotropium bromide, preferably tiotropium bromide monohydrate, and the lactose carrier, preferably lactose monohydrate.

## Patentansprüche

1. Verfahren zur Herstellung einer Trockenpulverformulierung zur Inhalation, die Tiotropiumbromid und einen Lactoseträger enthält, umfassend die Schritte:
a) Aufteilen des Lactoseträgers in eine erste, zweite und dritte Portion,
b) Mischen der ersten und zweiten Portion des Lactoseträgers mit dem Wirkstoff, indem
i) die erste Portion des Lactoseträgers in einen Mischer gegeben wird, um eine erste Schicht zu erhalten,
ii) der gesamte Wirkstoff auf die erste Lactoseschicht gegeben wird, um eine zweite Schicht zu erhalten,
iii) die zweite Portion des Lactoseträgers auf die zweite Schicht gegeben wird, um eine dritte Schicht zu erhalten,
iv) die im Schritt (iii) erhaltene Dreischichtzusammensetzung gemischt wird, um eine Vormischung zu erhalten,
v) gegebenenfalls die Vormischung gesiebt wird,
c) Mischen der im Schritt (b) erhaltenen Vormischung mit der dritten Portion des Lactoseträgers, um eine Trockenpulverformulierung zur Inhalation zu erhalten und
d) gegebenenfalls Sieben der Trockenpulverformulierung zur Inhalation.

2. Verfahren gemäß Anspruch 1, wobei der Lactoseträger durch Mischen einer grobkörnigen Lactose und feinkörnigen Lactose hergestellt wird.

3. Verfahren gemäß Anspruch 2, umfassend die Schritte:
a1) Aufteilen der grobkörnigen Lactose in eine erste, zweite und dritte Fraktion,
a2) Platzieren der ersten Fraktion der grobkörnigen Lactose in einen Mischer, um eine erste Schicht zu erhalten,
a3) Platzieren der gesamten feinkörnigen Lactose auf die erste Schicht, um eine zweite Schicht zu erhalten,
a4) Platzieren der zweiten Fraktion der grobkörnigen Lactose auf die zweite Schicht, um eine dritte Schicht zu erhalten,
a5) Mischen der im Schritt (a4) erhaltenen Dreischichtzusammensetzung, um eine Vormischung zu erhalten,
a6) gegebenenfalls Sieben der Vormischung,
a7) Mischen der im Schritt (a5) oder (a6) erhaltenen Vormischung mit der dritten Fraktion der grobkörnigen Lactose, um den Lactoseträger zu erhalten und
a8) gegebenenfalls Sieben des Lactoseträgers.

4. Verfahren gemäß Anspruch 3, wobei die erste, zweite und dritte Fraktion der grobkörnigen Lactose gleich sind.

5. Verfahren gemäß einem der Ansprüche 2-4, wobei der Gehalt an feinkörniger Lactose im Lactoseträger 30 Gew.-% oder weniger, vorzugsweise 10-30 Gew.-%, mehr bevorzugt 20-30 Gew.-% beträgt.

6. Verfahren gemäß einem der vorstehenden Ansprüche, wobei die Menge der ersten Portion, zweiten Portion und dritten Portion des Lactoseträgers jeweils mindestens 10 Gew.-%, bezogen auf das Gesamtgewicht des Lactoseträgers, beträgt.

7. Verfahren gemäß Anspruch 6, wobei die Menge der ersten Portion des Lactoseträgers 10-70 Gew.-%, die Menge der zweiten Portion des Lactoseträgers 10-20 Gew.-% und die Menge der dritten Portion des Lactoseträgers 10-70 Gew.-%, bezogen auf das Gesamtgewicht des Lactoseträgers, beträgt.

8. Verfahren gemäß Anspruch 6 oder 7, wobei das Gewichtsverhältnis der ersten Portion/zweiten Portion/dritten Portion des Lactoseträgers 7/2/1, 5/2/3, 5/1/4, 4/1/5 oder 2/1/7 ist.

9. Verfahren gemäß einen der vorstehenden Ansprüche, wobei die Trockenpulverformulierung zur Inhalation aus Tiotropiumbromid, vorzugsweise Tiotropiumbromid-Monohydrat, und den Lactoseträger, vorzugsweise Lactosemonohydrat, besteht.

## Revendications

1. Procédé de préparation d'une formulation d'inhalation de poudre sèche contenant du bromure de tiotropium et un porteur de lactose, comprenant les étapes consistant à :
a) diviser le porteur de lactose en une première, une deuxième et une troisième partie,
b) mélanger la première et la deuxième partie du porteur de lactose et le médicament en
i) plaçant la première partie du porteur de lactose dans un mélangeur pour obtenir une première couche,
ii) plaçant la totalité du médicament sur la première couche de lactose pour obtenir une seconde couche,
iii) plaçant la seconde partie du porteur de lactose sur la seconde couche pour obtenir une troisième couche,
iv) mélangeant la composition à trois couches obtenue à l'étape (iii) pour obtenir un prémélange,
v) tamiser facultativement le prémélange,
c) mélanger le prémélange obtenu à l'étape (b) et la troisième partie du porteur de lactose pour obtenir la formulation d'inhalation de poudre sèche, et
d) tamiser facultativement la formulation d'inhalation de poudre sèche.

2. Procédé selon la revendication 1, dans lequel le porteur de lactose est préparé en mélangeant du lactose grossier et du lactose fin.

3. Procédé selon la revendication 2, comprenant les étapes consistant à :
a1) diviser le lactose grossier en une première, une deuxième et une troisième fraction,
a2) placer la première fraction du lactose grossier dans un mélangeur pour obtenir une première couche,
a3) placer la totalité du lactose fin sur la première couche pour obtenir une deuxième couche,
a4) placer la deuxième fraction du lactose grossier sur la deuxième couche pour obtenir une troisième couche,
a5) mélanger la composition à trois couches obtenue à l'étape (a4) pour obtenir un prémélange,
a6) tamiser facultativement le prémélange,
a7) mélanger le prémélange obtenu à l'étape (a5) ou (a6) et la troisième fraction du lactose brut pour obtenir le porteur de lactose, et
a8) tamiser facultativement le porteur de lactose.

4. Procédé selon la revendication 3, dans lequel les première, deuxième et troisième fraction du lactose grossier sont égales.

5. Procédé selon l'une quelconque des revendications 2 à 4, dans lequel la teneur en lactose fin du porteur de lactose est de 30 % en poids ou moins, de préférence de 10 à 30 % en poids, de manière plus préférée de 20 à 30 % en poids.

6. Procédé selon l'une quelconque des revendications précédentes, dans lequel la quantité de la première partie, de la deuxième partie et de la troisième partie du porteur de lactose est d'au moins 10 % en poids, sur la base du poids total du porteur de lactose.

7. Procédé selon la revendication 6, dans lequel la quantité de la première partie du porteur de lactose est comprise entre 10 et 70 % en poids, la quantité de la deuxième partie du porteur de lactose est comprise entre 10 et 20 % en poids, et la quantité de la troisième partie du porteur de lactose est comprise entre 10 et 70 % en poids, par rapport au poids total du porteur de lactose.

8. Procédé selon la revendication 6 ou 7, dans lequel le rapport en poids de la première partie/deuxième partie/troisième partie du porteur de lactose est 7/2/1, 5/2/3, 5/1/4, 4/1/5 ou 2/1/7.

9. Procédé selon l'une quelconque des revendications précédentes, dans lequel la formulation d'inhalation de poudre sèche est constituée de bromure de tiotropium, de préférence de monohydrate de bromure de tiotropium, et du porteur de lactose, de préférence du monohydrate de lactose.
